# EUROPÄISCHE PATENTSCHRIFT

(11) **EP 1 916 998 B1**
(45) Veröffentlichungstag und Bekanntmachung des Hinweises auf die Patenterteilung: **26.02.2020**
(21) Anmeldenummer: 06776811.9
(22) Anmeldetag: 12.08.2006
(51) Int. Cl.: A61K 9/16, A23L 33/11, A23L 33/17, A23L 33/19

(54) **STEROLESTERPULVER**
STEROL ESTER POWDER
POUDRES D'ESTERS DE STEROLS

(30) Priorität: 23.08.2005 DE 102005039836
(43) Veröffentlichungstag der Anmeldung: 07.05.2008
(73) Patentinhaber: Cognis IP Management GmbH, 40589 Düsseldorf (DE)
(72) Erfinder: HORLACHER, Peter, 89287 Bellenberg (DE); GIERKE, Jürgen, 89257 Illertissen/Betlinshausen (DE); TIMMERMANN, Franz, 89257 Illertissen (DE); ALBIEZ, Wolfgang, 89257 Illertissen (DE); HOFMANN, Alois, 89257 Illertissen (DE)
(74) Vertreter: BASF IP Association
(86) Internationale Anmeldenummer: PCT/EP2006/007997
(87) Internationale Veröffentlichungsnummer: WO 2007/022890

(56) Entgegenhaltungen:
- WO-A-03/086108
- WO-A-2005/074717
- US-A- 6 113 972
- NGUYEN T T: "THE CHOLESTEROL-LOWERING ACTION OF PLANT STANOL ESTERS" JOURNAL OF NUTRITION, WISTAR INSTITUTE OF ANATOMY AND BIOLOGY, PHILADELPHIA, PA,, US, Bd. 129, Nr. 12, Dezember 1999 (1999-12), Seiten 2109-2112, XP008007936 ISSN: 0022-3166
- MENSINK R P ET AL: "EFFECTS OF PLANT STANOL ESTERS SUPPLIED IN LOW-FAT YOGHURT ON SERUM LIPIDS AND LIPOPROTEINS, NON-CHOLESTEROL STEROLS AND FAT SOLUBLE ANTIOXIDANT CONCENTRATIONS" ATHEROSCLEROSIS, AMSTERDAM, NL, Bd. 160, Nr. 1, Januar 2002 (2002-01), Seiten 205-213, XP001104063 ISSN: 0021-9150
- DATABASE WPI Week 200424 Derwent Publications Ltd., London, GB; AN 2004-252193 XP002410857 & JP 2004 075541 A (NIPPON OILS & FATS CO LTD) 11. März 2004 (2004-03-11)

## Beschreibung

### Gebiet der Erfindung

Die Erfindung befindet sich auf dem Gebiet der Lebensmittel und betrifft Formulierungen zur Einarbeitung in Lebensmittel, kosmetische und pharmazeutische Zubereitungen, die Sterol- und/oder Stanolester enthalten, ein Verfahren zur Herstellung derselben sowie Zubereitungen, insbesondere Lebensmittel, die diese Formulierungen beinhalten.

### Stand der Technik

Der Zusatz von Sterolen und Stanolen in Lebensmitteln aufgrund ihrer cholesterinsenkenden Eigenschaften und dadurch bedingten Prävention von Folgekrankheiten wie Atheriosklerose, Herzerkrankungen oder Bluthochdruck ist seit Jahren bekannt.

Da Phytosterole und -stanole in Wasser unlöslich und in Fetten und Ölen nur schlecht löslich sind, wirft die Einarbeitung der Cholesterinsenker in Lebensmittelzubereitungen, kosmetische oder pharmazeutische Produkte erhebliche Probleme auf. Aus dem ungünstigen Löslichkeitsverhalten der Substanzen resultiert neben der schlechten Dispergierbarkeit auch eine verminderte Bioverfügbarkeit und eine unbefriedigende Stabilität der Lebensmittelzubereitungen. Der Stand der Technik bietet daher zahlreiche Formulierungsvorschläge zur Lösung dieser Probleme an.

Zu den Bemühungen, die Löslichkeit zu erhöhen, gehörte die Formulierung von Estern der Sterole beispielsweise beansprucht in der Europäischen Patentanmeldung EP 1275309 A1 oder Estern der Stanole wie im US-Patent US 5,502,045 beschrieben, die zwar eine geringfügig verbesserte Verarbeitbarkeit, jedoch gegenüber den freien Sterolen eine geringere hypocholesterinämische Wirkung aufwiesen. Jedoch haben auch die veresterten Derivate noch ein unzureichendes Lösungsvermögen, um eine einfache Einarbeitung zu ermöglichen. Abgesehen davon, dass sich die veresterten Sterole in ihrer Bioverfügbarkeit anders verhalten, haben sie durch ihren unterschiedlichen physiko-chemischen Charakter auch andere Formulierungseigenschaften und erfordern den Einsatz anderer Hilfsstoffe.

In zahlreichen Patentanmeldungen wird beschrieben, wie die Verfügbarkeit von Sterolen über die Reduktion der Partikelgrößen vornehmlich durch Mikronisation verbessert werden kann. So beschreibt die deutsche Offenlegungsschrift DE 102 53 111 A1 pulverförmige Phytosterol-Formulierungen mit einer mittleren Teilchengröße von 0,01 bis 100 µm, die sich gut in Wasser redispergieren lassen. Einen Prozess zur Herstellung einer Steroldispersion, in der die Partikelgrößenverteilung der Sterole bei 0,1 bis 30 µm liegt entnimmt man der Internationalen Anmeldung WO 03/105611 A2**.**

Häufig ist jedoch die Mikronisation der Sterolpartikel alleine nicht ausreichend, um eine gute Einarbeitbarkeit zu erreichen. Zwar lässt sich durch die Erhöhung der Oberfläche die Bioverfügbarkeit der fein dispergierten Teilchen verbessern, jedoch sind gerade die mikronisierten Partikel schlecht benetzbar, aggregieren leicht und schwimmen auf wässrigen Oberflächen meistens auf. Häufig lässt sich das gemahlene Sterol nur mit speziellen Methoden in einem Getränk dispergieren, wozu intensives Mischen notwendig ist. Diese Geräte stehen jedoch im Normalfall dem Endanwender der Lebensmittelhersteller nicht zur Verfügung.

Daher kombinieren viele Hersteller die Mikronisation der Sterole mit der zusätzlichen Anwendung von Emulgatoren. Ein Beispiel dafür sind die in dem Europäischen Patent EP 0897671 B1 beanspruchten Zubereitungen mit Sterolen und Sterolestern mit einer Partikelgröße von maximal 15 µm in einem Gemisch mit ausgewählten Emulgatoren, wobei das Gewichtsverhältnis Emulgator zu Sterol in der wässrigen Phase weniger als 1:2 beträgt. Von speziellem Nachteil ist heir, dass die Formulierungen zu Agglomeration neigen.

Im Lebensmittelbereich häufig verwendete Emulgatoren sind Monoglyceride und Polysorbate **[**US 6,623,780 B1**,** US 6,376,482 B2**,** WO 02/28204 A1**].** In der Internationalen Patentanmeldung WO 03/086468 A1 werden pulverförmige Sterolesterformulierungen mit einem geringen Proteingehalt und ebenfalls Mono- und Diglyceriden als Emulgatoren offenbart. Auch wenn diese sich durch eine gute Verträglichkeit auszeichnen und als Lebensmittelemulgatoren über einen langen Zeitraum bereits bekannt sind, versucht man die Menge der Emulgatoren zu verringern oder sie gar ganz zu vermeiden, da Emulgatoren auch die Bioverfügbarkeit weiterer in den Lebensmitteln vorhandener Substanzen beeinflussen oder die Stabilität der Formulierungen negativ verändern können. Die Vermeidung von Emulgatoren war auch Ziel der in dem Europäischen Patent EP 1059851 B1 offenbarten Sterolformulierungen, die Verdicker zur besseren Dispergierbarkeit enthalten. Diese Zubereitungen erweisen sich indes als nicht ausschließlich stabil.

Zahlreiche weitere Methoden zur Verbesserung der Löslichkeit und Dispergierbarkeit wie die Formulierung als Emulsionen, Microemulsionen, Dispersionen, Suspensionen oder die Komplexierung mit Cyclodextrinen oder Gallensalzen werden in der Internationalen Patentanmeldung WO 99/63841 A1 vorgestellt, darunter auch die Formulierung in Form von Zubereitungen. Als Träger werden PEG, PVP, Copolymere, Celluloseether und -ester vorgeschlagen. Auch der direkte Einsatz von Lebensmittelgrundstoffen als Träger für pulverisierte Sterole ist aus der EP 1 003 388 B1 bekannt.

Die Auswahl von Proteinen als Trägersubstanzen für unveresterte Sterole und Stanole wird in der WO 01/37681 offenbart. Synergistische Effekte von Sitosterol und Sojaproteinen wurden im Europäischen Patent EP 0669835 B1 beschrieben. US 6113972 offenbart einen Phytosterol-Protein-Komplex enthaltend einen Proteingehalt von 20 bis 90 Gewichtsprozent bezogen auf den gesamten Komplex. Die Verwendung so geringer Mengen an Protein und so hoher Sterol/Stanolestermengen wie in der vorliegenden Erfindung offenbart ist nicht offenbart.WO 03/086108 offenbart Phytosterol-Emulsionen enthaltend Emulgatoren mit "niedrigem HLB-Wert wie Lecithin, destillierte Monoglyzeride..:". Solche Emulgatoren sind in vielen Anwendungen unerwünscht aufgrund der Deklarationspflicht auf fertigen Lebensmitteln, und daher von der vorliegenden Erfindung explizit ausgeschlossen sind.WO 2005/074717 offenbart partikulare Zusammensetzungen enthaltend wenigstens 1 Gewichtsprozent (als Sterol-Aquivalente, d.h. ggf. ohne das Gewicht der Sauren von Sterolestem zu berucksichtigen) Phytosterole. In der am meisten bevorzugten Ausfuhrungsform sind zwischen 10 und 40 Gewichtsprozent Phytosterolester (berechnet mit dem Gewicht der Sterolester, d.h. nicht als Sterol-Aquivalente) in den Zusammensetzungen enthalten. Diese tatsachlich erreichten Sterol/Stanolestermengen sind jedoch niedrig im Vergleich zu den sehr hohen Mengen von 62 bis 76 Gewichtsprozent Sterol-/Stanol-Fettsaureester in der vorliegenden Erfindung.JP2004/075541 A1 beansprucht partikulare Zusammensetzungen enthaltend Phytosterolester in breiten Mengenbereichen, offenbart aber tatsachlich keine Formulierungen, die denen der vorliegenden Erfindung nahekommen sowohl in konkreten Mengenverhaltnissen wie konkreter Zusammensetzung, beispielweise werden freie Phytosterole verwendet und keine Sterol/Stanolester.

Aufgabe der vorliegenden Erfindung war es, Formulierungen zur Verfügung zu stellen, die frei von den eingangs genannten Nachteilen sind und eine einfache und gute Dispergierung und Einarbeitung von Sterol- und/oder Stanolestern in Lebensmitteln ermöglichen, wobei die Anwendung von Emulgatoren speziell vom Typ der Lecithine, Monoglyceride, Diglyceride, Polysorbate, Natriumstearyllactylat, Glycerolmonostearat, Milchsäureester und Polyglycerinester vermieden werden soll. Diese Sterol- und/oder Sterolesterformulierungen sollten zudem einfach herzustellen sein und sich durch eine gute Lagerstabilität auszeichnen.

### Beschreibung der Erfindung

Gegenstand der Erfindung sind pulverförmige Zubereitungen gemäß Anspruch 1.

Die erfindungsgemäßen Formulierungen zeichnen sich infolge ihres Gehaltes an Milchpulver bzw. Proteinen, speziell an Emulgatoren vom Typ der Caseinate durch gute Solubilisierungseigenschaften, verminderte Aggregations- und Agglomerationseigenschaften und eine verbesserte Benetzbarkeit aus und ermöglichen so eine einfache Dispergierung von Sterol- und Stanolestern in wasser- und fetthaltigen Zubereitungen. Sie lassen sich hervorragend handhaben, da sie ohne aufwendige Ausrüstung weiterverarbeitet werden können und zeigen außerdem eine gute Lagerstabilität. Sie unterscheiden sich auf diese Weise vorteilhaft von solchen Zubereitungen, die entweder völlig frei von Emulgatoren sind oder Emulgatoren beispielsweise vom Typ der Lecithine, Monoglyceride, Diglyceride, Polysorbate, Natriumstearyllactylat, Glycerolmonostearat, Milchsäureester und Polyglycerinester aufweisen.

Die erfindungsgemäßen Zubereitungen können auf einfache Weise in Lebensmittel, insbesondere in Milch, Milchgetränke, Molke-, Joghurtgetränke, Margarine, Fruchtsäfte, Fruchtsaftgemische, Fruchtsaftgetränke, Gemüsesäfte, Kohlensäurehaltige und kohlensäurefreie Getränke, Sojamilchgetränke oder proteinreiche flüssige Nahrungsersatzgetränke, sowie fermentierte Milchzubereitungen, Joghurt, Trinkjoghurt, oder Käsezubereitungen, aber auch in kosmetische oder pharmazeutische Zubereitungen eingearbeitet werden.

Die Einschränkung "frei von anderen Emulgatoren als denen der Gruppe (b)" ist so zu verstehen, dass insbesondere der Einsatz von üblichen Lebensmittelemulgatoren wie Lecithinen, Monoglyceriden, Diglyceriden, Polysorbaten, Natriumstearyllactylat, Glycerolmonostearat, Milchsäureestern und Polyglycerinestern aus den oben eingangs geschilderten Gründen vermieden wird. Natürlich in den Lebensmitteln vorkommende Emulgatoren wie beispielsweise Cholesterol können selbstverständlich in der Preformulierung nicht verhindert werden, jedoch sollen während der Herstellung der erfindungsgemäßen Sterol-/Stanolesterzubereitungenen keine weiteren Emulgatoren als Hilfsstoffe zugefügt werden.

### Sterol- und/oder Stanolester

In der vorliegenden Erfindung werden Ester der aus Pflanzen und pflanzlichen Rohstoffen gewonnenen Sterole, sogenannte Phytosterole und Phytostanole eingesetzt. Bekannte Beispiele sind Ergosterol, Brassicasterol, Campesterol, Avenasterol, Desmosterol, Clionasterol, Stigmasterol, Poriferasterol, Chalinosterol, Sitosterol und deren Mischungen, darunter werden bevorzugt β- Sitosterol und Campesterol verwendet. Ebenso fallen die Ester der hydrierten gesättigten Formen der Sterole, der sogenannten Stanole unter die eingesetzten Verbindungen, auch hier werden β-Sitostanol- und Campestanolester bevorzugt. Als pflanzliche Rohstoffquellen dienen unter anderem Samen und Öle von Sojabohnen, Kanola, Palmkernen, Mais, Kokos, Raps, Zuckerrohr, Sonnenblume, Olive, Baumwolle, Soja, Erdnuss oder Produkte aus der Tallölproduktion. Es werden bevorzugt Veresterungsprodukte mit gesättigten und/oder ungesättigten Fettsäuren mit 6 bis 22 und vorzugsweise 12 bis 18 Kohlenstoffatomen verarbeitet. Die Erfindung ist jedoch nicht begrenzt auf diese Art der Ester. So sind auch Phenolsäureester, insbesondere Derivate der Zimtsäure, Kaffeesäure und Ferulasäure einsetzbar. Dabei können natürlich vorkommende Ester aus pflanzlichen Rohstoffen direkt gewonnen werden, oder die Sterol-/Stanolester werden durch Umesterung mit anderen Estern hergestellt. Ebenso können Derivate eingesetzt werden, die durch Veresterung freier Sterole oder Stanole mit den entsprechenden Fettsäuren entstanden sind. Die erfindungsgemäßen Zubereitungen enthalten 62 bis 76 Gew.-% Sterol- und/oder Stanolester bezogen auf die pulverförmigen Zubereitungen.

Ein weiterer Gegenstand der Erfindung betrifft daher Lebensmittelzubereitungen, die die Sterol-/Stanolesterformulierungen der genannten Zusammensetzung enthalten. Sie werden bevorzugt in Getränken und Milchprodukten eingesetzt, die dann 0,1 bis 50 Gew.-%, bevorzugt 1 bis 20 Gew.-% der pulverförmigen Zubereitungen bezogen auf das Gesamtgewicht der Lebensmittel, enthalten.

### Milchpulver und/oder Proteine

Als Milchpulver werden handelsübliche Vollmilch- und Magermilchpulver eingesetzt, die aus den jeweiligen Milchqualitäten durch Trocknung gewonnen worden sind. Sie können in Mischungen mit weiteren Proteinen oder als alleiniger Trägerstoff eingesetzt werden. Werden weitere Proteine zugesetzt oder Proteine anstelle des Milchpulvers als Trägerstoff verwendet, so sind darunter isolierte Proteine zu verstehen, die aus natürlichen tierischen und pflanzlichen Quellen gewonnen werden und bei der Herstellung der pulverförmigen Zubereitungen zugesetzt werden. Mögliche Quellen für Proteine stellen Pflanzen wie Weizen, Soja, Lupine, Mais oder Quellen tierischen Ursprungs wie Eier oder Milch dar. Vorzugsweise werden Milchpulver oder aus Milch gewonnene Proteine wie Natrium- und/oder Calciumcaseinate eingesetzt. Caseinate sind im Sinne der vorliegenden Erfindung besonders bevorzugt, da sie einerseits über emulgierende Eigenschaften verfügen ohne deshalb auch gleichzeitig die eingangs beschriebenen Nachteile solcher Lebensmittelemulgatoren zu zeigen, die sonst üblicherweise speziell für die Herstellung von Getränken und Milchprodukten, speziell Fermentationsprodukten wie Joghurt verwendet werden. Caseinate, speziell Natrium- oder Calciumcaseinate werden ansonsten üblicherweise als Emulgatoren zur Herstellung von Fleisch- und Wurstwaren verwendet.

Die erfindungsgemäßen Zubereitungen enthalten 12 bis 15 Gew.-% Milchpulver und/oder Proteine, vorzugsweise werden als Proteine Natriumcaseinat und/oder Calciumcaseinat bezogen auf die emulgatorfreie pulverförmige Zubereitung eingesetzt.

### Kohlenhydrate

Eine zusätzliche Verbesserung der Dispergierbarkeit, die insbesondere bei der Einarbeitung von Sterolesterpulvern in klare Flüssigkeiten Vorteile zeigt, konnte durch den Zusatz der weiteren Komponente c) in Form von Kohlenhydraten erreicht werden.
Die als Kohlenhydrate eingesetzten Verbindungen beinhalten alle lebensmittelgeeigneten Zucker, ausgewählt aus der Gruppe, die gebildet wird von Glucose, Saccharose, Fructose, Trehalose, Maltose, Maltodextrin, Cyclodextrin, Invertzucker, Palatinose und Lactose. Bevorzugt wird als Kohlenhydrat Glucose als Glucosesirup eingesetzt. Hinsichtlich der Dispergierbarkeit und Stabilität der Zubereitung hat sich der Einsatz von 10 bis 20 Gew.-% Kohlenhydrate bezogen auf das Gewicht der pulverförmigen Sterol/Stanolesterformulierung bewährt.

Besonders vorteilhaft in Bezug auf die Einarbeitbarkeit sind Formulierungen in denen der Anteil des Milchpulvers und/oder der Proteine - Komponente b) - zu den Kohlenhydraten - Komponente c) - in einem Gewichtsverhältnis von 1:5 bis 5:1, vorzugsweise 1:3 bis 3:1, besonders bevorzugt 1:1 bis 1 : 1,5 vorliegt.

### Weitere Hilfsstoffe

Als weitere Hilfsstoffe können die erfindungsgemäßen Zubereitungen Antioxidantien, Konservierungsmittel und Fließverbesserungsmittel enthalten. Beispiele für mögliche Antioxidantien oder Konservierungsmittel sind Tocopherole, Lecithine Ascorbinsäure, Parabene, Butylhydroxytoluol oder -anisol, Sorbinsäure oder Benzoesäure und deren Salze. Bevorzugt werden Tocopherole als Antioxidantien eingesetzt.
Als Fließregulierungs- und verbesserungsmittel soll bevorzugt Siliciumdioxid eingesetzt werden.

### Herstellung

Ein weiterer Gegenstand der Erfindung betrifft ein Verfahren zur Herstellung der pulverförmigen Sterol- und/oder Stanolesterzubereitungen, bei dem man
(i) Milchpulver und/oder Proteine (Komponente b) gegebenenfalls zusammen mit Antioxidantien und/oder Kohlenhydraten (Komponente c) in Wasser von Raumtemperatur dispergiert oder löst und auf 50 bis 90 °C erwärmt,
(ii) die ebenfalls auf 50 bis 90°C erwärmten Ester von Sterolen und/oder Stanolen (Komponente a) der erwärmten Dispersion zugibt und zu einer Emulsion homogenisiert, und schließlich
(iii) die entstandene Emulsion sprühtrocknet.

Vorzugsweise erhält man die emulgatorfreien pulverförmigen Zubereitungen dadurch, dass man
(i) das Milchpulver und/oder die Proteine (Komponente b) gegebenenfalls zusammen mit Antioxidantien und/oder Kohlenhydraten (Komponente c) in Wasser von Raumtemperatur dispergiert oder löst und auf 60 bis 80 °C erwärmt,
(ii) die ebenfalls auf 60 bis 80°C erwärmten Ester von Sterolen und/oder Stanolen (Komponente a) der erwärmten Dispersion zugibt und zu einer Emulsion homogenisiert, und
(iii) die entstandene Emulsion wird über eine rotierende Scheibe fein zerteilt und sprühgetrocknet.

### Pulverförmige Zubereitungen

Folgende Zubereitungen sind (alle Angaben jeweils bezogen auf das Gesamtgewicht der jeweiligen Zubereitung) vorteilhaft jedoch nicht von der vorliegenden Erfidung beansprucht:
(A) Pulverförmige Zubereitungen, enthaltend
   (a) 50 bis 90 Gew.-% Ester von Sterolen und/oder Stanolen und
   (b) 5 bis 50 Gew.-% Milchpulver.
(B) Pulverförmige Zubereitungen, enthaltend
   (a) 50 bis 90 Gew.-% Fettsäureester von Sterolen und/oder Stanolen und
   (b) 10 bis 30 Gew.-% Milchpulver und/oder Proteine.

   Aufgrund der verbesserten Lagerstabilität, Dispergierbarkeit und Verarbeitbarkeit in Lebensmitteln zeigen die folgenden Rezepturen gute Eigenschaften:
(C) Pulverförmige Zubereitungen, enthaltend
   (a) 60 bis 80 Gew.-% Fettsäureester von Sterolen und/oder Stanolen und
   (b) 5 bis 50 Gew.-% Milchpulver und/oder Proteine.
D) Pulverförmige Zubereitungen, enthaltend
   (a) 50 bis 90 Gew.-% Fettsäureester von Sterolen und/oder Stanolen,
   (b) 5 bis 50 Gew.-% Milchpulver und/oder Proteine und
   (c) 3 bis 30 Gew.-% Kohlenhydrate.

   Sehr gute Eigenschaften zeigen die Zubereitungen in der folgenden Zusammensetzung:
(E) Pulverförmige Zubereitungen, enthaltend
   (a) 60 bis 80 Gew.-% Fettsäureester von Sterolen und/oder Stanolen und
   (b) 10 bis 30 Gew.-% Milchpulver und/oder Natrium- und/oder Calciumcaseinat.
(F) Pulverförmige Zubereitungen, enthaltend
   (a) 60 bis 80 Gew.-% Fettsäureester von Sterolen und/oder Stanolen,
   (b) 12 bis 20 Gew.-% Milchpulver und/oder Proteine und
   (c) 5 bis 20 Gew.-% Kohlenhydrate.
(G) Pulverförmige Zubereitungen, enthaltend
   (a) 60 bis 80 Gew.-% Fettsäureester von Sterolen und/oder Stanolen,
   (b) 10 bis 30 Gew.-% Milchpulver und/oder Natrium- und/oder Calciumcaseinat und
   (c) 10 bis 20 Gew.-% Kohlenhydrate.

   Besonders gute Eigenschaften zeigen die Emulsionen in der folgenden Zusammensetzung:
(H) Pulverförmige Zubereitungen, enthaltend
   (a) 62 bis 76 Gew.-% Fettsäureester von Sterolen und/oder Stanolen und
   (b) 10 bis 30 Gew.-% Milchpulver und/oder Natrium- und/oder Calciumcaseinat.
(I) Pulverförmige Zubereitungen, enthaltend
   (a) 60 bis 80 Gew.-% Fettsäureester von Sterolen und/oder Stanolen,
   (b) 5 bis 50 Gew.-% Natrium- und/oder Calciumcaseinat und
   (c) 10 bis 20 Gew.-% Glucose.
(J) Pulverförmige Zubereitungen, enthaltend
   (a) 60 bis 80 Gew.-% Fettsäureester von Sterolen und/oder Stanolen,
   (b) 10 bis 30 Gew.-% Natrium- und/oder Calciumcaseinat und
   (c) 3 bis 30 Gew.-% Glucose

Die beste Lagerstabilität wiesen Formulierungen der folgenden erfindungsgemäßen Zusammensetzung auf:

Pulverförmige Zubereitungen bestehend aus bezogen auf das Gesamtgewicht der Zubereitung (a) 62 bis 76 Gew.-% Ester von Sterolen und/oder Stanolen (b) 12 bis 15 Gew.-% Milchpulver und/oder Proteine und (c) 10 bis 20 Gew.-% Kohlenhydrate, die ausgewählt sind aus der Gruppe, die zusammengesetzt ist aus Glucose, Saccharose, Fructose, Trehalose, Maltose, Maltodextrin, Cyclodextrin, Invertzucker, Palatinose und Lactose, (d) optional weitere Hilfsstoffe, mit der Massgabe, dass sich die Mengenangaben der Substanzen (a) bis (d) zu 100 Gew.% ergänzen, und dass keine anderen Emulgatoren als die der Gruppe b) enthalten sind.

### Beispiele

### Beispiel 1

29,5 g Sprühmagermilchpulver (Fa. Almil), 0,5 g Tetrakaliumpyrophosphat (Chem. Fa. Budenheim) und 0,05 g Natriumascorbat (Fa. Rewe) wurden in 170 g Wasser dispergiert. Die Dispersion wurde auf ca. 70 °C erhitzt. 70 g Sterolester (Vegapure® 95 E - Cognis Deutschland GmbH & Co. KG) wurden auf ca. 70 °C erwärmt und unter Rühren zu der wässrigen Dispersion gegeben. Der pH-Wert der Dispersion lag zwischen 6,8 und 7,4. Die so hergestellte Voremulsion wurde anschließend im Homogenisator Typ LAB 100/60 der Firma Schröder bei 220/30bar im Kreislauf ungefähr drei Durchgänge homogenisiert, wobei die Emulsionstemperatur auf 65 bis 70°C gehalten wurde. Die so hergestellte Emulsion wurde schließlich im APV Sprühturm Typ LAB 3 S unter folgenden Bedingungen versprüht:

| | |
|---|---|
| Trockensubstanz | : ca. 45 % |
| Zulufttemperatur | : 180°C |
| Ablufttemperatur | : 90+/-5°C |
| Zerstäuberdrehzahl | : 24000 Upm |

**Fließbett**

| | |
|---|---|
| Zulufttemperatur Sektion ½ | : 30-35°C |
| Zulufttemperatur Sektion 3 | : 4 bis 8 °C |
| Rotationssieb | : 1 mm |

In nachfolgenden Mischern (Fa. Gericke) wurden 1 % SiO₂ als Fließhilfsmittel zugegeben. Das so hergestellte Pulver zeichnete sich bei Raumtemperatur durch eine gute Einrührbarkeit sowohl in Wasser als auch in Milch (0,3 und 1,5 Gew.-% Fettanteil) aus.

### Beispiel 2

14,8 g Casein (Fa. Almil), wurden in 75 g Wasser dispergiert und mit ca. 9 g 20%iger Natronlauge versetzt, auf 80°C erwärmt und gerührt bis eine klare Lösung mit einem pH-Weret von ca. 7 entstanden war. Anschließend wurden 12 g Glucosesirup (80%) und 0,05 g Natriumascorbat (Fa. Rewe) in 100 g Wasser dispergiert. Die Dispersion wurde auf ca. 70 °C erhitzt und zur Na-Caseinat-lösung gegeben. 71 g Sterolester (Vegapure® 95 E - Cognis Deutschland GmbH & Co. KG) wurden auf ca. 70 °C erwärmt und unter Rühren zu der wässrigen Dispersion gegeben. Der pH-Wert der Dispersion lag zwischen 6,8 und 7,4. Diese Voremulsion wurde im Homogenisator Typ LAB 100/60 der Firma Schröder bei 220/30bar im Kreislauf ungefähr drei Durchgänge homogenisiert, wobei die Emulsionstemperatur auf 65 bis 70°C gehalten wurde. Die so hergestellte Emulsion wurde schließlich im APV Sprühturm Typ LAB 3 S wie unter Beispiel 1) beschrieben gesprüht.

## Patentansprüche

1. Pulverförmige Zubereitungen bestehend aus bezogen auf das Gesamtgewicht der Zubereitung
(a) 62 bis 76 Gew.-% Ester von Sterolen und/oder Stanolen
(b) 12 bis 15 Gew.-% Milchpulver und/oder Proteine und
(c) 10 bis 20 Gew.-% Kohlenhydrate, die ausgewählt sind aus der Gruppe, die zusammengesetzt ist aus Glucose, Saccharose, Fructose, Trehalose, Maltose, Maltodextrin, Cyclodextrin, Invertzucker, Palatinose und Lactose,
(d) optional weitere Hilfsstoffe,
mit der Massgabe, dass sich die Mengenangaben der Substanzen (a) bis (d) zu 100 Gew.-% ergänzen, und dass keine anderen Emulgatoren als die der Gruppe b) enthalten sind.

2. Pulverförmige Zubereitungen nach Anspruch 1, **dadurch gekennzeichnet, dass** die Ester der Gruppe a) Fettsäureester mit 6 bis 22 Kohlenstoffatmen sind.

3. Pulverförmige Zubereitungen nach Anspruch 1 oder 2, **dadurch gekennzeichnet, dass** sie bezogen auf das Gesamtgewicht der Zubereitung als Komponente (b) Natrium und/oder Calciumcaseinat enthalten.

4. Pulverförmige Zubereitungen nach einem der Ansprüche 1 bis 3, **dadurch gekennzeichnet, dass** sie bezogen auf das Gesamtgewicht er Zubereitung als Komponente (c) Glucose enthalten.

5. Pulverförmige Zubereitungen nach einem der Ansprüche 1 bis 4, wobei als weitere Hilfsstoffe Antioxidantien, Konservierungsmittel und/oder Fliessverbesserer enthalten sind.

6. Pulverförmige Zubereitungen nach Anspruch 5, wobei als Antioxidantien Tocopherole enthalten sind.

7. Pulverförmige Zubereitungen nach Anspruch 5 oder 6, wobei als Fliessverbesserer Siliziumdioxid enthalten ist.

8. Pulverförmige Zubereitungen nach einem der Ansprüche 1 bis 7, wobei die Anteile von Komponente (b) und Komponente (c) in einem Gewichtsverhältnis von 1:5 bis 5:1 stehen.

9. Pulverförmige Zubereitungen nach Anspruch 8, wobei die Anteile von Komponente (b) und Komponente (c) in einem Gewichtsverhältnis von 1:3 bis 3:1 stehen.

10. Pulverförmige Zubereitungen nach Anspruch 8, wobei die Anteile von Komponente (b) und Komponente (c) in einem Gewichtsverhältnis von 1:1 bis 1:1,5 stehen.

11. Lebensmittel, enthaltend 0,1 bis 50 Gew.-% der pulverförmigen Zubereitungen nach einem der Ansprüche 1 bis 10.

12. Getränke und Milchprodukte, enthaltend 0,1 bis 50 Gew.-% der pulverförmigen Zubereitung nach einem der Ansprüche 1 bis 11.

13. Verfahren zur Herstellung von pulverförmigen Sterol- bzw. Stanolesterzubereitungen nach einem der Ansprüche 1 bis 10, bei dem man
(i) Komponente b) gegebenenfalls zusammen mit optionaler Komponente d), bevorzugt Antioxidantien, und Komponente c) in Wasser von Raumtemperatur dispergiert oder löst und auf 50 bis 90 °C erwärmt,
(ii) die ebenfalls auf 50 bis 90°C erwärmten Ester von Sterolen und/oder Stanolen (Komponente a) der erwärmten Dispersion oder Lösung zugibt und zu einer Emulsion homogenisiert und anschließend
(iii) die entstandene Emulsion sprühtrocknet.

## Claims

1. A pulverulent preparation consisting of, based on the total weight of the preparation,
(a) 62 to 76% by weight esters of sterols and/or stanols
(b) 12 to 15% by weight milk powder and/or proteins and
(c) 10 to 20% by weight carbohydrates selected from the group which is composed of glucose, sucrose, fructose, trehalose, maltose, maltodextrin, cyclodextrin, invert sugar, palatinose and lactose,
(d) optionally further auxiliaries,
with the proviso that the amounts of the substances (a) to (d) add up to 100% by weight, and that no emulsifiers are present other than those of group (b) .

2. The pulverulent preparation according to claim 1, wherein the esters of group (a) are fatty acid esters having 6 to 22 carbon atoms.

3. The pulverulent preparation according to claim 1 or 2, wherein said preparation comprises sodium and/or calcium caseinate as component (b), based on the total weight of the composition.

4. The pulverulent preparation according to any of claims 1 to 3, wherein said preparation comprises glucose as component (c), based on the total weight of the composition.

5. The pulverulent preparation according to any of claims 1 to 4, wherein antioxidants, preservatives and/or flow improvers are present as further auxiliaries.

6. The pulverulent preparation according to claim 5, wherein tocopherols are present as antioxidants.

7. The pulverulent preparation according to claim 5 or 6, wherein silicon dioxide is present as flow improver.

8. The pulverulent preparation according to any of claims 1 to 7, wherein the proportions of component (b) and component (c) are in a ratio by weight from 1:5 to 5:1.

9. The pulverulent preparation according to claim 8, wherein the proportions of component (b) and component (c) are in a ratio by weight from 1:3 to 3:1.

10. The pulverulent preparation according to claim 8, wherein the proportions of component (b) and component (c) are in a ratio by weight from 1:1 to 1:1.5.

11. A foodstuff comprising 0.1 to 50% by weight of the pulverulent formulations according to any of claims 1 to 10.

12. A drink or a milk product comprising 0.1 to 50% by weight of the pulverulent formulation according to any of claims 1 to 11.

13. A process for producing pulverulent sterol ester preparations or stanol ester preparations according to any of claims 1 to 10, wherein
(i) component b) optionally together with optional component d), preferably antioxidants, and component c) are dispersed or dissolved in water at room temperature and heated to 50 to 90°C,
(ii) the esters of sterols and/or stanols (component a), also heated to 50 to 90°C, are added to the heated dispersion or solution and homogenized to give an emulsion and then
(iii) the resulting emulsion is spray-dried.

## Revendications

1. Préparations sous forme de poudre constituées de, par rapport au poids total de la préparation
(a) 62 à 76 % en poids d'ester de stérols et/ou de stanols
(b) 12 à 15 % en poids de poudre de lait et/ou de protéines et
(c) 10 à 20 % en poids de glucides, qui sont choisis dans le groupe, qui est composé du glucose, du saccharose, du fructose, du tréhalose, du maltose, de la maltodextrine, de la cyclodextrine, du sucre inverti, du palatinose et du lactose,
(d) éventuellement d'autres adjuvants,
étant entendu que les données quantitatives des substances (a) à (d) se complètent jusqu'à 100 % en poids, et qu'aucuns autres émulsifiants que ceux du groupe b) ne sont contenus.

2. Préparations sous forme de poudre selon la revendication 1, **caractérisées en ce que** les esters du groupe a) sont des esters d'acide gras comportant 6 à 22 atomes de carbone.

3. Préparations sous forme de poudre selon la revendication 1 ou 2, **caractérisées en ce qu'**elles contiennent du caséinate de sodium et/ou du caséinate de calcium en tant que composant b), par rapport au poids total de la préparation.

4. Préparations sous forme de poudre selon l'une quelconque des revendications 1 à 3, **caractérisées en ce qu'**elles contiennent du glucose en tant que composant (c), par rapport au poids total de la préparation.

5. Préparations sous forme de poudre selon l'une quelconque des revendications 1 à 4, des antioxydants, des agents de conservation et/ou des agents d'amélioration de l'écoulement sont contenus en tant qu'autres adjuvants.

6. Préparations sous forme de poudre selon la revendication 5, des tocophérols étant contenus en tant qu'antioxydants.

7. Préparations sous forme de poudre selon la revendication 5 ou 6, du dioxyde de silicium étant contenu en tant qu'agent d'amélioration de l'écoulement.

8. Préparations sous forme de poudre selon l'une quelconque des revendications 1 à 7, les proportions en composant (b) et en composant (c) se situant dans un rapport en poids de 1:5 à 5:1.

9. Préparations sous forme de poudre selon la revendication 8, les proportions en composant (b) et en composant (c) se situant dans un rapport en poids de 1:3 à 3:1.

10. Préparations sous forme de poudre selon la revendication 8, les proportions en composant (b) et en composant (c) se situant dans un rapport en poids de 1:1 à 1:1,5.

11. Produit alimentaire, contenant 0,1 à 50 % en poids des préparations sous forme de poudre selon l'une quelconque des revendications 1 à 10.

12. Boissons et produits à base de lait, contenant 0,1 à 50 % en poids de la préparation sous forme de poudre selon l'une quelconque des revendications 1 à 11.

13. Procédé pour la fabrication de préparations sous forme de poudre d'ester de stérol respectivement de stanol selon l'une quelconque des revendications 1 à 10, dans lequel
(i) on disperse ou on dissout dans de l'eau à température ambiante, le composant b), éventuellement ensemble avec le composant d) éventuel, préférablement des antioxydants, et le composant c), et on chauffe à une température de 50 à 90 °C,
(ii) on ajoute les esters de stérols et ou de stanols (composant a), également chauffés à une température de 50 à 90 °C, à la dispersion ou à la solution chauffée, et on homogénéise en une émulsion et par la suite
(iii) on sèche par pulvérisation l'émulsion obtenue.
